(19) Europäisches Patentamt
European Patent Office
Office européen des brevets

(11) Veröffentlichungsnummer: **0 129 050**
**A1**

(12) **EUROPÄISCHE PATENTANMELDUNG**

(21) Anmeldenummer: 84105439.8

(22) Anmeldetag: 14.05.84

(51) Int. Cl.³: C 07 C 79/46
A 01 N 37/48

(30) Priorität: 27.05.83 DE 3319287

(43) Veröffentlichungstag der Anmeldung:
27.12.84 Patentblatt 84/52

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Förster, Heinz, Dr.
Am Eckbusch 47
D-5600 Wuppertal 1(DE)

(72) Erfinder: Gallenkamp, Bernd, Dr.
Claudiusweg 5
D-5600 Wuppertal 1(DE)

(72) Erfinder: Priesnitz, Uwe, Dr.
Severinstrasse 58
D-5650 Solingen 1(DE)

(72) Erfinder: Riebel, Hans-Jochem, Dr.
In der Beek 92
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert R., Dr.
Im Waldwinkel 110
D-5060 Bergisch Gladbach 2(DE)

(54) **Optisch aktive Diphenylether-Derivate.**

(57) Neue R-Enantiomere von Diphenylether-Derivaten der Formel

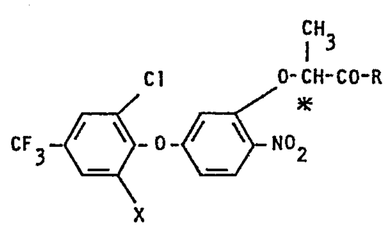

(I)

in welcher
X für Wasserstoff oder Halogen steht und
R für Hydroxy oder Alkoxy steht,
mehrere Verfahren zur Herstellung dieser neuen Stoffe und deren Verwendung als Herbizide.

EP 0 129 050 A1

0129050

BAYER AKTIENGESELLSCHAFT 5090 Leverkusen, Bayerwerk

Konzernverwaltung RP
Patentabteilung Dü/m-c
Ib

Optisch aktive Diphenylether-Derivate

Die Erfindung betrifft neue R-Enantiomere*) von Diphenylether-Derivaten, mehrere Verfahren zu deren Herstellung und deren Verwendung als Herbizide.

Es ist bereits bekannt, daß zahlreiche Diphenylether-Derivate herbizide Eigenschaften besitzen (vgl. US-PS 4 093 446 und DE-OS 23 11 638). So können zum Beispiel die Racemate des 2-/3-(2-Chlor-4-trifluormethyl-phenoxy)-6-nitro-phenoxy/-propionsäure-ethylesters und des 2-/3-(2,6-Dichlor-4-trifluormethyl-phenoxy)-6-nitro-phenoxy/-propionsäure-ethylesters zur Bekämpfung von Unkraut eingesetzt werden. Die Wirkung dieser Stoffe ist jedoch nicht immer ausreichend.

---

*) Unter R-Enantiomeren sind hier jeweils diejenigen optisch aktiven Verbindungen zu verstehen, welche am asymmetrischen Kohlenstoffatom die R-Konfiguration aufweisen.

Es wurden jetzt neue R-Enantiomere von Diphenylether-Derivaten der Formel

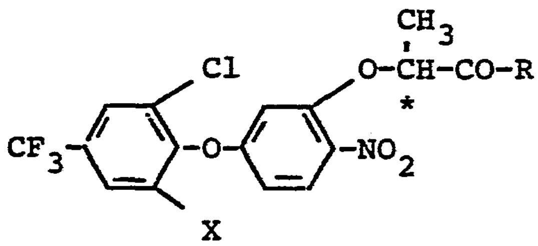

(I)

in welcher

X für Wasserstoff oder Halogen steht und

R für Hydroxy oder Alkoxy steht,

gefunden.

Weiterhin wurde gefunden, daß man die neuen R-Enantiomeren von Diphenylether-Derivaten der Formel (I) erhält, wenn man

a) Diphenylether der Formel

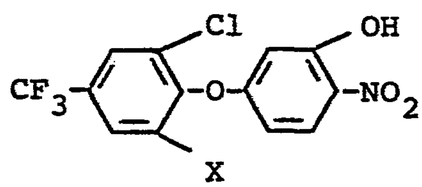

(II)

in welcher

X die oben angegebene Bedeutung hat,

mit S-Enantiomeren der Propionsäure-Derivate der Formel

$$Z - \underset{*}{\overset{CH_3}{CH}} - \overset{O}{\overset{\|}{C}} - O - R^1 \qquad (III)$$

in welcher

$R^1$ für Alkyl steht und

Z für Tosylat oder Mesylat steht,

gegebenenfalls in Gegenwart eines Säureakzeptors sowie gegebenenfalls in Gegenwart eines Verdünnungsmittels umsetzt, und gegebenenfalls die dabei entstehenden Ester in Gegenwart eines Verdünnungsmittels verseift,

oder

b) Diphenylether-Derivate der Formel

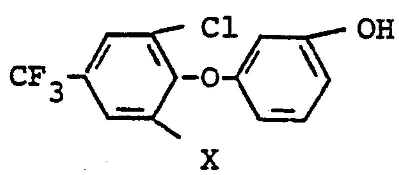

(IV)

in welcher

X die oben angegebene Bedeutung hat

mit S-Enantiomeren der Propionsäure-Derivate der Formel

$$Z - \underset{*}{\overset{CH_3}{CH}} - \overset{O}{\overset{\|}{C}} - O - R^1 \qquad (III)$$

in welcher

$R^1$ für Alkyl steht und

Z für Tosylat oder Mesylat steht,

gegebenenfalls in Gegenwart eines Säureakzeptors sowie gegebenenfalls in Gegenwart eines Verdünnungsmittels umsetzt und anschließend die dabei entstehenden R-Enantiomeren der Diphenylether der Formel

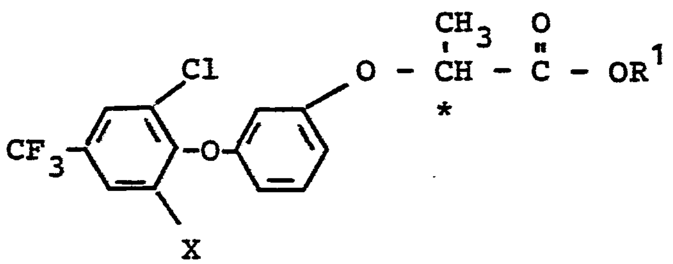

(V)

in welcher

$R^1$ und X die oben angegebene Bedeutung haben,

mit einem Nitrierungsmittel, gegebenenfalls in Gegenwart eines Katalysators sowie gegebenenfalls in Gegenwart eines Verdünnungsmittels umsetzt und gegebenenfalls die dabei entstehenden Ester in Gegenwart eines Verdünnungsmittels verseift.

Schließlich wurde gefunden, daß sich die neuen R-Enantiomeren von Diphenylether-Derivaten der Formel (I) durch eine hervorragende herbizide Wirksamkeit auszeichnen.

Überraschenderweise besitzen die erfindungsgemäßen R-Enantiomeren der Diphenylether-Derivate der Formel (I) wesentlich bessere herbizide Eigenschaften als die Racemate des 2-/3-(2-Chlor-4-trifluormethyl-phenoxy)-6-nitro-phenoxy7-propionsäure-ethylesters und des 2-/3-(2,6-Dichlor-4-trifluormethyl-phenoxy)-6-nitro-phenoxy7-propionsäure-ethylesters, die aus dem Stand der Technik als gut wirksame Herbizide bekannt sind.

Die erfindungsgemäßen optisch aktiven Verbindungen sind durch die Formel (I) allgemein definiert. In dieser Formel, in der das asymmetrische Kohlenstoffatom durch ein (*) gekennzeichnet ist, steht X vorzugsweise für Wasserstoff oder Chlor. R steht vorzugsweise für Hydroxy oder für geradkettiges oder verzweigtes Alkoxy mit 1 bis 4 Kohlenstoffatomen, insbesondere für Methoxy oder Ethoxy.

Verwendet man 2-Chlor-4-trifluormethyl-3'-hydroxy-4'-nitro-diphenylether und das S-Enantiomere des 2-Tosyloxy-propionsäure-ethylesters als Ausgangsstoffe, so kann der Verlauf des erfindungsgemäßen Verfahrens (a) durch das folgende Formelschema wiedergegeben werden:

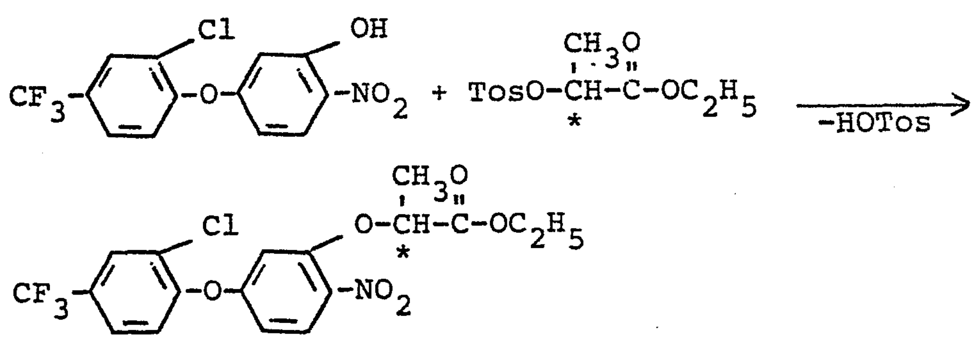

Tos = 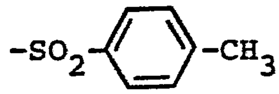 (= Tosyl)

Verwendet man 2,6-Dichlor-4-trifluormethyl-3'-hydroxy-diphenylether und das S-Enantiomere des 2-Tosyloxy-propionsäure-ethylesters als Ausgangsstoffe und konzentrierte Salpetersäure als Nitrierungsmittel, so kann der Verlauf des erfindungsgemäßen Verfahrens (b) durch das folgende Formelschema wiedergegeben werden:

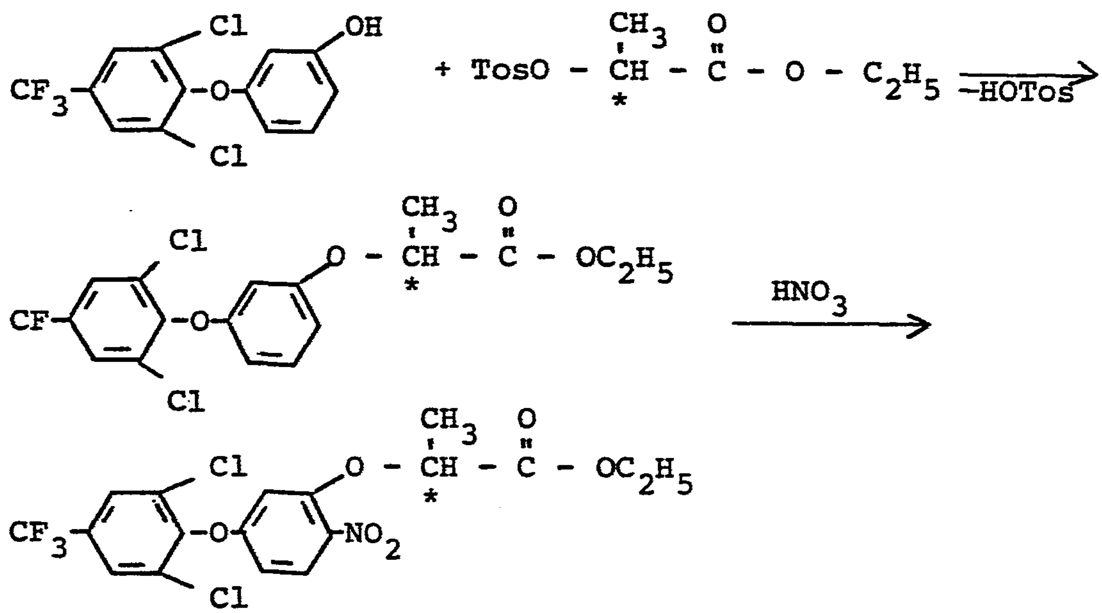


Die bei dem erfindungsgemäßen Verfahren (a) als Ausgangsstoffe benötigten Diphenylether sind durch die Formel (II) definiert. In dieser Formel steht X vorzugsweise für Wasserstoff oder Chlor.

Die Diphenylether der Formel (II) sind bereits bekannt (vgl. DE-OS 29 06 087).

Die bei dem erfindungsgemäßen Verfahren (a) weiterhin als Ausgangsstoffe benötigten S-Enantiomeren der Propionsäure-Derivate sind durch die Formel (III) definiert. In dieser Formel steht $R^1$ vorzugsweise für geradkettiges oder verzweigtes Alkyl mit 1 bis 4 Kohlenstoffatomen, insbesondere für Methyl oder Ethyl. Z steht für Tosylat ($-O-SO_2-$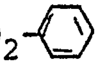$-CH_3$) oder Mesylat ($-O-SO_2-CH_3$).

Die S-Enantiomeren der Propionsäure-Derivate der Formel (III) sind bekannt oder lassen sich nach bekannten Methoden in einfacher Weise herstellen.

Bei der Herstellung der R-Enantiomeren der Diphenylether-Derivate der Formel (I) nach dem erfindungsgemäßen Verfahren (a) ist der Einsatz von S-Enantiomeren der Propionsäure-Derivate der Formel (III) erforderlich, da im Verlauf der Umsetzung am asymmetrischen Kohlenstoffatom eine Walden'sche Umkehr erfolgt. Die S-Enantiomeren der Propionsäure-Derivate der Formel (III) drehen die Ebene des linear polarisierten Lichtes nach links. Den gleichen Drehsinn besitzen die erfindungsgemäßen Stoffe, obwohl sich die Konfiguration am asymmetrischen Kohlenstoffatom im Zuge der Herstellung umgekehrt hat.

Als Säureakzeptoren können bei den erfindungsgemäßen Verfahren (a) alle üblicherweise für derartige Umsetzungen

verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische und heterocyclische Amine, beispielsweise Triethylamin, Trimethylamin, Dimethylanilin, Dimethylbenzylamin, 1,5-Diazabicyclo/4,3,0/non-5-en (DBN), 1,8-Diazabicyclo/5,4,0/undec-7-en (DBU) und Pyridin.

Als Verdünnungsmittel kommen bei der Durchführung des erfindungsgemäßen Verfahrens (a) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Umsetzung nach dem erfindungsgemäßen Verfahren (a) innerhalb eines

größeren Bereiches variiert werden. Im allgemeinen arbeitet man bei Temperaturen zwischen -20°C und +160°C, vorzugsweise zwischen 0°C und +140°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren. Im allgemeinen geht man so vor, daß man das Reaktionsgemisch mit Wasser versetzt, mehrfach mit einem mit Wasser wenig mischbaren organischen Lösungsmittel extrahiert, die vereinigten organischen Phasen nacheinander mit verdünnter wäßriger Alkalilauge und Wasser wäscht, dann trocknet und durch Abziehen des Lösungsmittels unter vermindertem Druck einengt.

Ist die Herstellung einer Verbindung der Formel (I) beabsichtigt, in der R für Hydroxy steht, so ist eine Verseifung der zunächst entstehenden Ester erforderlich. Diese Verseifung erfolgt nach üblichen Methoden. Vorzugsweise verwendet man wäßrige Alkalihydroxidlaugen, wie zum Beispiel Natronlauge oder Kalilauge, als Verseifungsreagenzien.

Die Esterverseifung wird im allgemeinen in Gegenwart eines Verdünnungsmittels vorgenommen. Vorzugsweise in Betracht kommen aromatische Kohlenwasserstoffe, wie Toluol oder Xylol, ferner Alkohole, wie Methanol oder Ethanol, außerdem Ether, wie Dioxan, weiterhin Nitrile, wie Acetonitril, und auch Gemische aus organischen Solventien und Wasser.

Die Temperaturen können bei der Durchführung der Esterverseifung innerhalb eines größeren Bereiches variiert werden. Im allgemeinen arbeitet man bei Temperaturen zwischen 0°C und 160°C, vorzugsweise zwischen 20°C und 140°C.

Bei der Durchführung der Esterverseifung geht man im allgemeinen so vor, daß man den Ester der Formel (I) in Gegenwart eines Verdünnungsmittels mit einem Überschuß an Base bei der jeweils gewünschten Temperatur behandelt. Die Aufarbeitung erfolgt nach üblichen Methoden. Im allgemeinen geht man so vor, daß das Reaktionsgemisch durch Abziehen des Lösungsmittels unter vermindertem Druck einengt, den verbliebenen Rückstand in Wasser aufnimmt, mit Mineralsäure, wie zum Beispiel Salzsäure, ansäuert und die sich dabei abscheidende Säure der Formel (I) abtrennt.

Die bei der Durchführung des erfindungsgemäßen Verfahrens (b) als Ausgangsstoffe benötigten Diphenylether-Derivate sind durch die Formel (IV) definiert. In dieser Formel steht X vorzugsweise für Wasserstoff oder Chlor.

Die Diphenylether-Derivate der Formel (IV) sind bekannt (vgl. DE-OS 28 05 981).

Die bei dem erfindungsgemäßen Verfahren (b) weiterhin als Ausgangsstoffe benötigten S-Enantiomeren der Propionsäure-Derivate der Formel (III) wurden bereits im Zusammenhang mit der Beschreibung des erfindungsgemäßen Verfahrens (a) erwähnt. Auch in diesem Fall ist der Einsatz von S-Enantiomeren der Verbindungen der Formel (III) erforderlich, da im Verlauf der Reaktion eine Walden'sche Umkehr am asymmetrischen Kohlenstoffatom erfolgt.

Zur Nitrierung der R-Enantiomeren der Diphenylether der Formel (V) verwendet man im Falle des erfindungsgemäßen Verfahrens (b) vorzugsweise konzentrierte Salpetersäure oder Schwermetall-Nitrate, wie zum Beispiel Kupfernitrat oder Eisen(III)nitrat.

Die erste Stufe des erfindungsgemäßen Verfahrens (b) wird vorzugsweise in Gegenwart eines Säureakzeptors und eines Verdünnungsmittels durchgeführt. Hierbei kommen als Säurebindemittel und Verdünnungsmittel alle diejenigen Stoffe in Betracht, die bereits im Zusammenhang mit der Beschreibung des erfindungsgemäßen Verfahrens (a) genannt wurden.

Als Katalysatoren für die in der zweiten Stufe des erfindungsgemäßen Verfahrens (b) durchzuführende Nitrierung kommen vorzugsweise Protonensäuren, wie zum Beispiel Schwefelsäure oder Essigsäure, in Betracht.

Als Verdünnungsmittel kommen bei der Durchführung der zweiten Stufe des erfindungsgemäßen Verfahrens (b) vorzugsweise halogenierte Kohlenwasserstoffe, wie zum Beispiel Methylenchlorid, und ferner Essigsäureanhydrid in Frage.

Die Reaktionstemperaturen können bei dem erfindungsgemäßen Verfahren (b) innerhalb eines größeren Bereiches variiert werden. Im allgemeinen arbeitet man in der ersten Stufe bei Temperaturen zwischen -20°C und +160°C, vorzugsweise zwischen 0°C und +140°C. In der zweiten Stufe arbeitet man im allgemeinen bei Temperaturen zwischen -20°C und +140°C, vorzugsweise zwischen 0°C und +100°C.

Das erfindungsgemäße Verfahren (b) wird im allgemeinen unter Normaldruck durchgeführt. Es ist jedoch auch möglich, unter erhöhtem oder vermindertem Druck zu arbeiten.

Die Durchführung des erfindungsgemäßen Verfahrens (b) erfolgt in der ersten Stufe so, wie es bereits für das erfindungsgemäße Verfahren (a) beschrieben wurde. Zur Durchführung der zweiten Stufe des erfindungsgemäßen Verfahrens (b) setzt man auf 1 Mol einer Verbindung der Formel (V) im allgemeinen 0,9 bis 2 Mol, vorzugsweise

1,0 bis 1,3 Mol an Nitrierungsmittel und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden. Im allgemeinen verfährt man in der Weise, daß man das Reaktionsgemisch nach beendeter Umsetzung in Eiswasser gießt, absaugt und das anfallende Produkt gegebenenfalls durch Umkristallisieren reinigt.

Wenn nach dem erfindungsgemäßen Verfahren (b) diejenigen Verbindungen der Formel (I) hergestellt werden sollen, in denen R für Hydroxy steht, dann führt man die Ester-Verseifung so durch, wie es bereits im Falle des erfindungsgemäßen Verfahrens (a) beschrieben wurde.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

 

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine,

z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

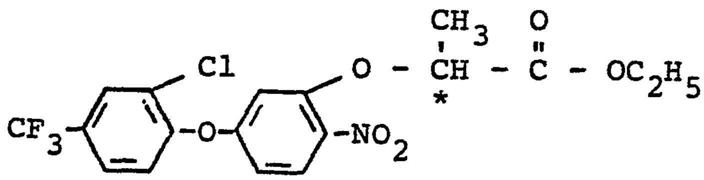

Verfahrensvariante (a):

Ein Gemisch aus 33,4 g (0,1 Mol) 2-Chlor-4-trifluormethyl-3'-hydroxy-4'-nitro-diphenylether, 28,5 g (0,1 Mol) an S-Enantiomerem des 2-Tosyloxy-propionsäure-ethylesters und 28 g (0,2 Mol) Kaliumcarbonat in 200 ml Acetonitril wurde 15 Stunden unter Rückfluß erhitzt. Danach wurde aufgearbeitet, indem man das Reaktionsgemisch in Wasser gab, mehrfach mit Toluol extrahierte, die vereinigten organischen Phasen nacheinander mit Wasser, verdünnter wäßriger Natronlauge und wieder mit Wasser wusch, dann trocknete und durch Abziehen des Lösungsmittels unter vermindertem Druck einengte. Der verbleibende Rückstand wurde durch leichtes Erwärmen im Hochvakuum von Resten an flüchtigen Anteilen befreit. Man erhielt auf diese Weise 33 g (76 % der Theorie) an R-Enantiomerem des 2-[3-(2-Chlor-4-trifluormethyl-phenoxy)-6-nitro-phenoxy]-propionsäure-ethylesters.

Drehwert: $[\alpha]_D^{24}$ = -12,7°

(1-molare Lösung in Chloroform; Kuvettenlänge 10 cm)

Verfahrensvariante (b):

Ein Gemisch aus 19,9 g (0,07 Mol) 2-Chlor-4-trifluormethyl-3'-hydroxy-diphenylether, 19,7 g des S-Enantiomeren des 2-Tosyloxy-propionsäure-ethylesters und 9,7 g Kaliumcarbonat in 100 ml Acetonitril wurde 15 Stunden unter Rückfluß erhitzt. Danach wurde das Reaktionsgemisch auf Raumtemperatur abgekühlt, mit 400 ml Wasser versetzt und dann zweimal mit je 200 ml Toluol extrahiert. Die vereinigten organischen Phasen wurden über Natriumsulfat getrocknet und durch Abziehen des Lösungsmittels unter vermindertem Druck eingeengt. Der verbleibende Rückstand wurde durch leichtes Erwärmen im Hochvakuum von Resten an flüchtigen Anteilen befreit. Man erhielt auf diese Weise 14 g an R-Enantiomerem des 2-[3-(2-Chlor-4-trifluormethyl-phenoxy)-phenoxy]-propionsäure-ethylesters.

$n_D^{20}$ = 1,5171

Drehwert: $[\alpha]_D^{24}$ = + 3,65°

(1-molare Lösung in Chloroform; Küvettenlänge 10 cm)

In eine Mischung von 16 ml Essigsäureanhydrid und 4,7 g Kupfernitrat [$Cu(NO_3)_2 \cdot 3H_2O$] wurden innerhalb von 30 Minuten 13,1 g (0,034 Mol) des R-Enantiomeren des 2-[3-(2-Chlor-4-trifluormethyl-phenoxy)-phenoxy]-propionsäureethylesters unter Rühren so eingetropft, daß die Temperatur des Reaktionsgemisches +35°C nicht überstieg. Nach dreistündigem Rühren bei Raumtemperatur wurden noch einmal 0,47 g Kupfernitrat [$Cu(NO_3)_2$ . 3 $H_2O$] hinzugefügt. Es wurde 12 Stunden bei Raumtemperatur nachgerührt. Danach arbeitete man auf, indem man das Reaktionsgemisch in 150 ml Eiswasser goß, das entstehende Gemisch mehrfach mit Ether extrahierte, die vereinigten organischen Phasen trocknete und unter vermindertem Druck einengte. Man erhielt auf diese Weise 13 g (88 % der Theorie) an R-Enantiomerem des 2-[3-(2-Chlor-4-trifluormethyl-phenoxy)-6-nitro-phenoxy]-propionsäure-ethylesters.

Drehwert: $[\alpha]_D^{24}$ = -11,0°

(1-molare Lösung in Chloroform; Küvettenlänge 10 cm).

Beispiel 2

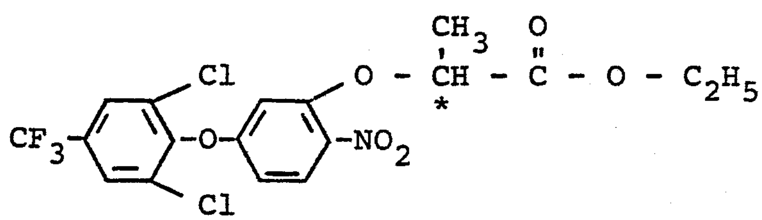

Nach der im Beispiel 1 angegebenen Verfahrensvariante (a) wurde auch das R-Enantiomere des 2-[3-(2,6-Dichlor-4-trifluormethyl-phenoxy)-6-nitro-phenoxy]-propionsäure-ethylesters hergestellt.

Ausbeute: 22 g (47 % der Theorie)

Drehwert: $[\alpha]_D^{24}$ = -14,4°

(1-molare Lösung in Chloroform; Küvettenlänge 10 cm).

Beispiel 3

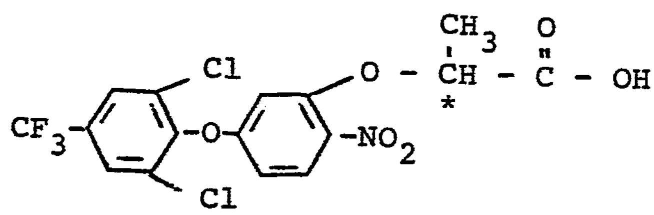

Eine Lösung von 17,6 g (0,44 Mol) Natriumhydroxid in 25 ml Wasser wurde unter schnellem Rühren in eine auf 120°C erhitzte Lösung von 206 g (0,44 Mol) des R-Enantiomeren des 2-[3-(2,6-Dichlor-4-trifluormethyl-phenoxy)]-propionsäure-ethylesters in 500 ml Xylol langsam eingetropft. Man rührte noch weitere 1,5 Stunden bei 120°C und arbeitete dann auf, indem man das Lösungsmittel abdestillierte, den verbleibenden Rückstand mit Wasser aufnahm, dann mit 50 ml konzentrierter Salzsäure ansäuerte und das sich abscheidende Produkt abtrennte. Man erhielt auf diese Weise 70 g (36,2 % der Theorie) an R-Enantiomerem der 2-[3-(2,6-Dichlor-4-trifluormethyl-phenoxy)-6-nitro-phenoxy]-propionsäure.

Drehwert: $[\alpha]_D^{24}$ = -20,4°

(1-molare Lösung in Chloroform; Küvettenlänge 10 cm)

Beispiel 4

Herstellung des Ausgangsproduktes der Formel

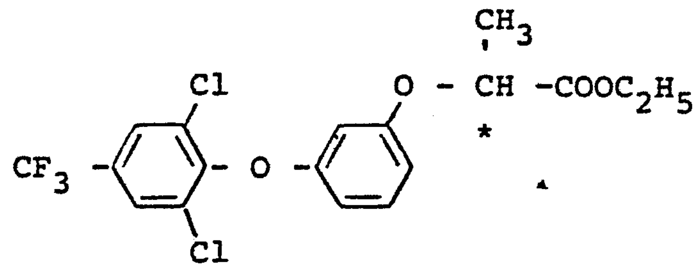

(V-2)

Ein Gemisch aus 96,9 g (0,3 Mol) 2,6-Dichlor-4-trifluormethyl-3'-hydroxy-diphenylether, 81,7 g (0,3 Mol) des S-Enantiomeren des 2-Tosyloxy-propionsäure-ethylesters und 82,8 g (0,6 Mol) Kaliumcarbonat in 800 ml Acetonitril wurde 3 Stunden unter Rückfluß erhitzt. Danach wurde aufgearbeitet, indem man das Reaktionsgemisch absaugte, das Filtrat einengte, den verbleibenden Rückstand in Methylenchlorid löste, die entstehende Lösung einmal mit Wasser wusch und nach dem Trocknen unter vermindertem Druck einengte. Der verbleibende Rückstand wurde durch leichtes Erwärmen im Hochvakuum von Resten an flüchtigen Anteilen befreit. Man erhielt auf diese Weise das R-Enantiomere des 2-/3-(2,6-Dichlor-4-trifluormethyl-phenoxy)-phenoxy/-propionsäure-ethylesters in einer Ausbeute von 97 % der Theorie.

Drehwert: $[\alpha]_D^{24}$ = + 1,5°

(1-molare Lösung in Chloroform; Küvettenlänge 10 cm).

In den folgenden Verwendungsbeispielen wurden die nachstehend aufgeführten Verbindungen eingesetzt:

(A) = 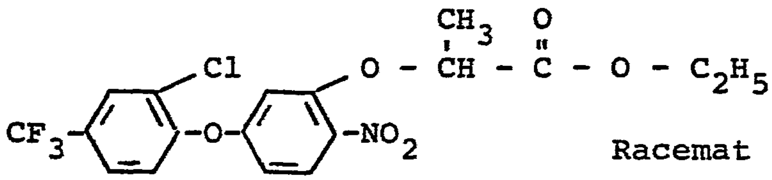

Racemat

(bekannt aus DE-OS 23 11 638)

(B) = 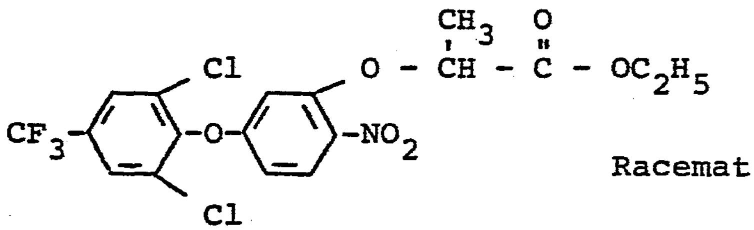

Racemat

(bekannt aus DE-OS 23 11 638)

(1) = 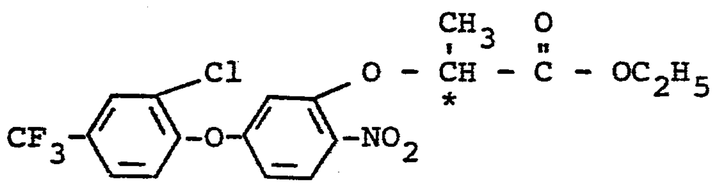

R-Enantiomeres

(2) = 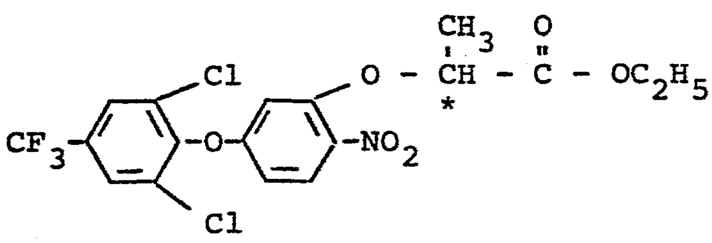

R-Enantiomeres

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Wirkstoffkonzentrationen und Versuchsergebnisse gehen aus der folgenden Tabelle hervor:

## Tabelle A

Pre-emergence-Test

| Wirkstoff | Aufwandmenge in kg/ha | % Wirkung bei Dicotyledoneae | Monocotyledoneae | Rüben | Soya | Mais |
|---|---|---|---|---|---|---|
| (A) | 1 | 95 | 70,7 | 99 | 30 | 0 |
| (1) | 0,61 | 98,4 | 92 | 99 | 30 | 10 |
| (B) | 1 | 98,4 | 80,7 | 100 | 40 | 0 |
| (2) | 0,48 | 99,6 | 87,9 | 100 | 45 | 0 |

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Wirkstoffe, Wirkstoffkonzentrationen und Versuchsergebnisse gehen aus der folgenden Tabelle hervor:

**T a b e l l e  B**

**Post-emergence-Test**

| Wirkstoff | Aufwandmenge in kg/ha | % Wirkung bei Dicotyledoneae | Monocotyledoneae | Rüben | Soya | Mais |
|---|---|---|---|---|---|---|
| (A) | 1 | 100 | 97,6 | 100 | 100 | 100 |
| (1) | 0,61 | 99,9 | 98,1 | 100 | 100 | 99 |
| (B) | 1 | 99,9 | 98,9 | 100 | 100 | 100 |
| (2) | 0,48 | 100 | 99 | 100 | 100 | 100 |

Patentansprüche:

1. R-Enantiomere von Diphenylether-Derivaten der Formel

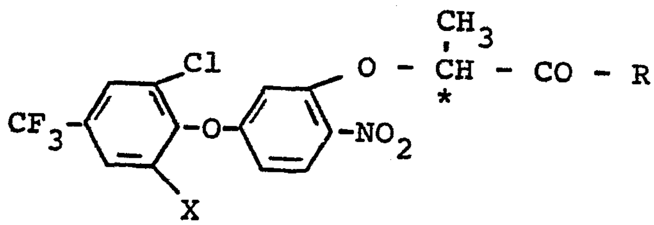

(I)

in welcher

X für Wasserstoff oder Halogen steht und

R für Hydroxy oder Alkoxy steht.

2. R-Enantiomere von Diphenylether-Derivaten der Formel (I), in denen

X für Wasserstoff oder Chlor steht und

R für Hydroxy oder Alkoxy mit 1 bis 4 Kohlenstoffatomen steht.

3. Verfahren zur Herstellung von R-Enantiomeren von Diphenylether-Derivaten der Formel

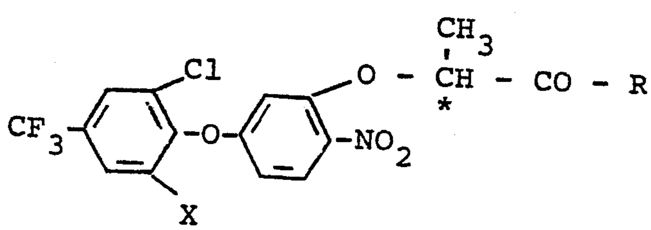

(I)

in welcher

X für Wasserstoff oder Halogen steht und

R für Hydroxy oder Alkoxy steht,

dadurch gekennzeichnet, daß man

a) Diphenylether der Formel

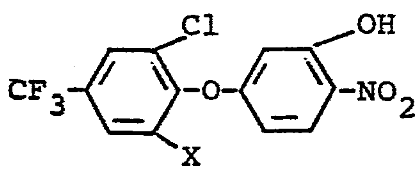

(II)

in welcher

X die oben angegebene Bedeutung hat,

mit S-Enantiomeren der Propionsäure-Derivate der Formel

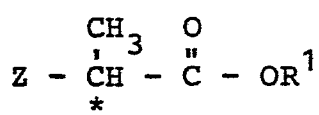

(III)

in welcher

$R^1$ für Alkyl steht und

Z für Tosylat oder Mesylat steht,

gegebenenfalls in Gegenwart eines Säureakzeptors sowie gegebenenfalls in Gegenwart eines

Verdünnungsmittels umsetzt, und gegebenenfalls die dabei entstehenden Ester in Gegenwart eines Verdünnungsmittels verseift,

oder

b) Diphenylether-Derivate der Formel

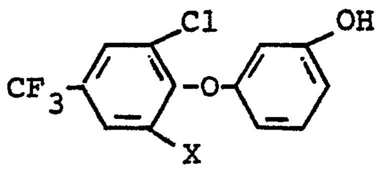

(IV)

in welcher

X die oben angegebene Bedeutung hat

mit S-Enantiomeren der Propionsäure-Derivate der Formel

$$Z - \underset{*}{\overset{\overset{CH_3}{|}}{CH}} - \overset{\overset{O}{\|}}{C} - O - R^1 \qquad (III)$$

in welcher

$R^1$ für Alkyl steht und

Z für Tosylat oder Mesylat steht,

gegebenenfalls in Gegenwart eines Säureakzeptors sowie gegebenenfalls in Gegenwart eines Verdünnungsmittels umsetzt und anschließend die dabei entstehenden R-Enantiomeren der Diphenylether der Formel

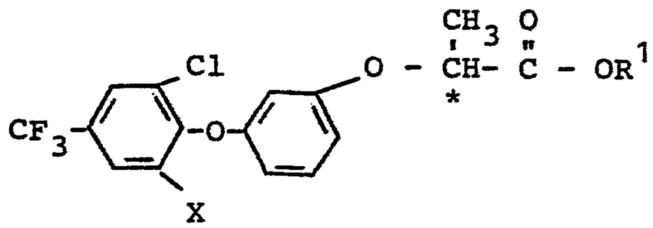

(V)

in welcher

$R^1$ und X die oben angegebene Bedeutung haben,

mit einem Nitrierungsmittel, gegebenenfalls in Gegenwart eines Katalysators sowie gegebenenfalls in Gegenwart eines Verdünnungsmittel umsetzt und gegebenenfalls die dabei entstehenden Ester in Gegenwart eines Verdünnungsmittels verseift.

4. Herbizide Mittel, gekennzeichnet durch einen Gehalt an mindestens einem R-Enantiomeren eines Diphenylether-Derivates der Formel (I).

5. Verwendung von R-Enantiomeren von Diphenylether-Derivaten der Formel (I) als Herbizide.

6. Verfahren zur Bekämpfung von Unkräutern, dadurch gekennzeichnet, daß man R-Enantiomere von Diphenylether-Derivaten der Formel (I) auf die Unkräuter und/oder deren Lebensraum ausbringt.

7. Verfahren zur Herstellung von herbiziden Mitteln, dadurch gekennzeichnet, daß man R-Enantiomere von Diphenylether-Derivaten der Formel (I) mit Streckmitteln und/oder oberflächenaktiven Stoffen vermischt.

8) R-Enantiomeres des Diphenylether-Derivates der Formel

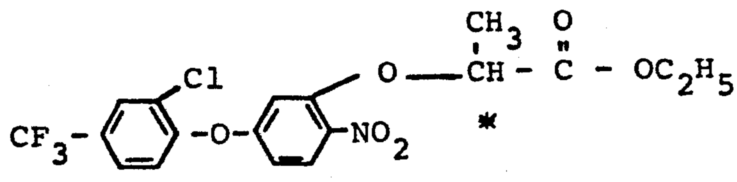

(R)

9) R-Enantiomeres des Diphenylether-Derivates der Formel

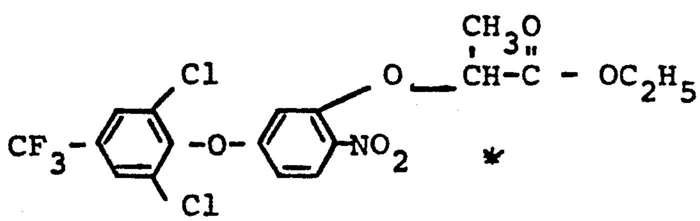

(R)

1o) R-Enantiomeres des Diphenylether Derivates der Formel

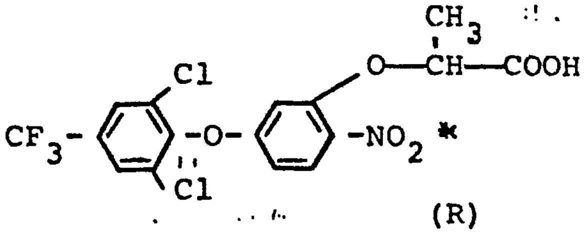

(R)

0129050

Europäisches Patentamt

# EUROPÄISCHER RECHERCHENBERICHT

Nummer der Anmeldung

EP 84 10 5439

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | DE-A-2 311 638 (ROHM & HAAS)<br>* Ansprüche 1-4, 10-11, 15,17,18, 21-24; Seite 35; Beispiel 50 *<br>--- | 1,2,4-10 | C 07 C 79/46<br>A 01 N 37/48 |
| Y | EP-A-0 025 079 (HOFFMANN-LA ROCHE)<br>* Ansprüche 1-12 *<br>--- | 1,4-6 | |
| A | EP-A-0 009 285 (AKZO)<br>* Ansprüche 1-3 *<br>----- | 1,4-6 | |
| | | | RECHERCHIERTE SACHGEBIETE (Int. Cl. 3) |
| | | | C 07 C 79/00<br>C 07 C 59/00<br>C 07 C 51/00<br>C 07 C 69/00 |

Der vorliegende Recherchenbericht wurde für alle Patentansprüche erstellt.

| Recherchenort | Abschlußdatum der Recherche | Prüfer |
|---|---|---|
| DEN HAAG | 31-08-1984 | KLAG M.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument